# EUROPÄISCHE PATENTSCHRIFT

(11) **EP 1 465 575 B1**
(45) Veröffentlichungstag und Bekanntmachung des Hinweises auf die Patenterteilung: **05.06.2013**
(21) Anmeldenummer: 02747283.6
(22) Anmeldetag: 16.04.2002
(51) Int. Cl.: A61J 1/05, A61M 1/16

(54) **EINSCHWEISSSCHIFFCHEN FÜR EINEN BEUTEL**
WELDING SHUTTLE FOR A BAG
NAVETTE SOUDABLE POUR UN SAC

(30) Priorität: 17.01.2002 DE 20200689 U
(43) Veröffentlichungstag der Anmeldung: 13.10.2004
(73) Patentinhaber: Fresenius Medical Care Deutschland GmbH, 61352 Bad Homburg (DE)
(72) Erfinder: BRANDL, Matthias, 97631 Bad Königshofen (DE); HILGERS, Peter, 97453 Schonungen (DE); KUGELMANN, Franz, 66606 St. Wendel/Bliesen (DE); MEISINGER, Matthias, 66583 Spiesen/Elversberg (DE)
(74) Vertreter: Laufhütte, Dieter
(86) Internationale Anmeldenummer: PCT/EP2002/004221
(87) Internationale Veröffentlichungsnummer: WO 2003/059241

(56) Entgegenhaltungen:
- DE-A- 3 305 365
- DE-A- 3 803 776
- DE-A- 19 958 952
- DE-C- 19 634 944
- DE-C- 19 959 230
- US-A- 5 823 383

## Beschreibung

Die Erfindung betrifft ein Einschweiß-Schiffchen nach dem Oberbegriff des Anspruchs 1.

Aus der DE 201 08 911 U ist bereits ein Aufbewahrungsbeutel mit einem aus einem Einschweiß-Schiffchen bestehenden Portsystem zur Entnahme und Zugabe bekannt. Hier ist beutelseitig ein Ventilsystem vorhanden, welches beim Verbinden mit dem Entnahme- oder Zugabeteil geöffnet wird und beim Trennen selbsttätig schließt. Die Ports sind dort als Luer-Konus ausgebildet.

Auch aus der DE 33 05 365 C2 ist ein Aufbewahrungsbeutel mit Einschweiß-Schiffchen bekannt, das einen Mittelteil und zwei sich von diesem aus in entgegengesetzte Richtung erstreckende spitz zulaufende Fortsätze aufweist. Dieses weist bereits mittig eine Öffnung zum Befüllen des Beutels mit einer Substanz bei der Herstellung und andererseits Zu- und Abgänge für die eigentliche Benutzung des Beutels auf. Bei den bekannten Aufbewahrungsbeuteln ist die Einzelöffnung jedoch nur mit einem sehr engen Lumen ausgebildet, wodurch die Befüllung erschwert wird.

Die DE 199 58 952 A1 offenbart ein Entnahmesystem für medizinische Lösungen, das ein in den Beutel einschweißbares Basisteil, das ein Entnahme- oder Zuspritzteil beinhaltet, umfasst. Der Entnahme- und Zuspritzteil ist im wesentlichen mit der Außenseite des Basisteils bündig verbunden.

Aufgabe der Erfindung ist es, einen Beutel mit einem Einschweiß-Schiffchen zu schaffen, wobei der Herstellungsprozess des Beutels vereinfacht und gleichzeitig die Handhabung durch den Benutzer erleichtert werden soll.

Erfindungsgemäß wird diese Aufgabe dadurch gelöst, daß ein gattungsgemäßes Einschweiß-Schiffchen zusätzlich die kennzeichnenden Merkmale des Anspruchs 1 aufweist. Demnach ist das Mittelteil im wesentlichen durch eine möglichst großlumige und vorzugsweise kreisrunde Öffnung ausgefüllt, wobei die Seitenkanten der seitlichen Fortsätze sich im Bereich, in dem sie Spitz zulaufen, tangential an die Öffnung anlegen. Damit wird ein Einschweiß-Schiffchen für flexible Behältnisse wie Beutel mit platzoptimierter Anordnung geschaffen, die ein vereinfachtes Füllen des Beutels ermöglichen. Der Anschlußport, d.h. die Öffnung zum Befüllen des Beutels ist durch eine zentrale, formstabile Öffnung gekennzeichnet, über die der Beutel als solches sehr leicht und insbesondere schnell gefüllt werden und anschließend verschlossen werden kann. Aufgrund einer symmetrischen Geometrie des Einschweiß-Schiffchens, nämlich die seitlich sich tangential an die Öffnung anlegenden Fortsätze, ist Platz für weitere funktionelle Komponenten wie den Zugabe- und Entnahmeport oder eventuell anzuschließende Evakuierungsports neben der zentralen Öffnung zum Befüllen des Beutels gegeben.

Vorteilhafte Ausführungsformen der Erfindung ergeben sich aus den sich an den Hauptanspruch anschließenden Unteransprüchen.

Demnach ist an der großlumigen Öffnung des Einschweiß-Schiffchens gemäß einer bevorzugten Ausgestaltung eine Tülle angesetzt, vorzugsweise angespritzt. Über diese Tülle kann der mit dem Einschweiß-Schiffchen versehene Beutel während des Befüllens einfach fixiert werden. Damit eignet sich das derartig weitergebildete Einschweiß-Schiffchen insbesondere zum Einsatz in einer automatischen Produktionslinie zum Befüllen der mit dem Einschweiß-Schiffchen versehenen Beutel.

Im Bereich der Fortsätze des Einschweiß-Schiffchens können vorteilhaft die Ports bzw. Konnektoren je nach Bedarf angeordnet sein, zum Beispiel ein Zugabeport auf der einen Seite und ein Entnahmeport auf der anderen.

Besonders vorteilhaft sind am Umfang des Einschweiß-Schiffchens sogenannte Anschweißkanten vorgesehen. Entlang dieser Kanten läßt sich der flexible Beutel über eine Schweißverbindung in einfacherer Weise stoffschlüssig mit dem Einschweiß-Schiffchen verbinden. Diese Anschweißkanten können auch nur zur Toleranzaufnahme vorgesehen sein, während ansonsten die Schweißung auf der gesamten Seitenfläche vorgenommen wird.

Im Rahmen der Erfindung wird zusätzlich für einen Beutel Schutz beansprucht, der ein vorzugsweise pulverförmiges Konzentrat für eine Dialyselösung oder von Teilen davon zur Nierenersatzbehandlung enthält und der ein Einschweiß-Schiffchen nach den vorgenannten Ansprüchen aufweist.

In vorzugsweiser Ausgestaltung kann dieser Beutel ein Einschweiß-Schiffchen aufweisen, das zwei Ports enthält. Ein Port kann dabei insbesondere als Einströmöffnung über ein Rohr in den Beute, der andere insbesondere als Ausströmöffnung in den Beutel münden. Beide Ports sind vorteilhafterweise nach außen ragend ausgeführt. An den Enden der Ports können sich Vorrichtungen zur Aufnahme funktioneller Komponenten wie Filter oder Schläuche befinden. Es ist auch möglich, einen Port über ein Rohr und/oder Schlauch bis zum tiefsten Punkt des Beutels zu führen, um einen gezielten Zugang zu diesem Bereich zu haben - sei es für Zugabe - oder Entnahmezwecke.,

Weitere Einzelheiten und Vorteile werden anhand eines in der Zeichnung dargestellten Ausführungsbeispiels erläutert. Es zeigen:
- Fig. 1:: eine Draufsicht auf ein Einschweiß-Schiffchen gemäß einem Ausführungsbeispiel der vorliegenden Erfindung,
- Fig. 2:: eine geschnittene Seitenansicht des Einschweiß-Schiffchen nach Figur 1,
- Fig. 3:: eine perspektivische Darstellung des Einschweiß-Schiffchens, das im wesentlichen den Ansichten nach Figur 1 bzw. Figur 2 entspricht und
- Figur 4:: eine geschnittene Seitenansicht eines Beutels mit einem erfindungsgemäßen Einschweiß-Schiffchen.

In der in den Figuren dargestellten Ausführungsvariante der Erfindung wird der grundlegende Aufbau eines Einschweiß-Schiffchen beschrieben, das beispielsweise für einen Konzentratbeutel zur Zubereitung von Dialyseflüssigkeit eingesetzt wird.

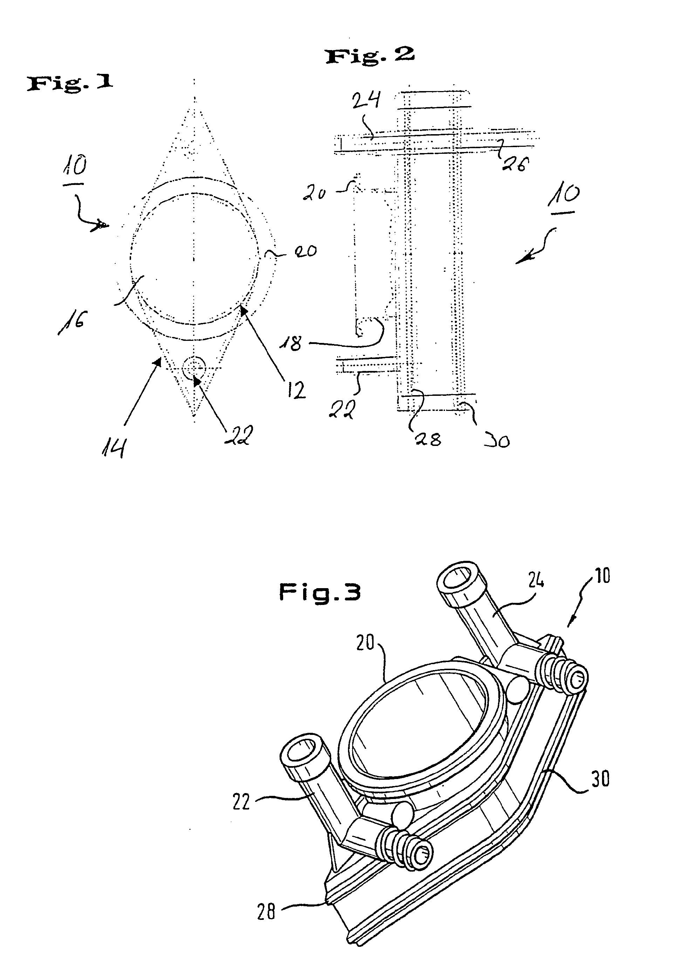

Das Einschweiß-Schiffchen 10 besteht aus einem Mittelteil 12 und aus in entgegengesetze Richtung verlaufenden und spitz zulaufenden Fortsätzen 14, die untereinander symmetrisch ausgebildet sind. Wie insbesondere aus der Figur 1 zu erkennen ist, ist das Mittelteil 12 im wesentlichen durch eine großlumige, hier im Ausführungsbeispiel 35 mm Durchmesser aufweisende, und im vorliegenden Ausführungsbeispiel kreisrunde Öffnung 16 ausgefüllt. Diese Befüllöffnung ermöglicht ein schnelles und einfaches Einfüllen von Konzentratpulver. Wie aus der Figur 1 und 3 deutlich wird, verlaufen die Seitenkanten der seitlichen Fortsätze derart, daß sie sich tangential an die Seitenwandung der Öffnung 16 anlegen. Hierdurch wird also eine Platzoptimierung unter Erreichen einer großlumigen Öffnung 16 zum Befüllen gewährleistet.

Aus den Figuren 2 und 3 wird deutich, daß an die Öffnung 16 eine Tülle 18 angespritzt ist, an deren freien Ende ein Flansch 20 ausgebildet ist. Diese Tülle erleichtert einerseits die Positionierung eines hier nicht näher dargestellten Füllrohrs einer Befüllmaschine. Andererseits kann durch Aufstülpen oder Aufschweißen eines Deckels bzw. einer Folie die Öffnung 16 in einfacher Art und Weise verschlossen werden.

Im Bereich der Fortsätze 14 sind Ports 22 bzw. 24 angeordnet. Diese Anschlußports 22 und 24 können in hier nicht näher dargestellter Art und Weise beispielsweise unmittelbar mit einem Wasserkreislauf verbunden sein. Während der Port 22 hier auf dem Einschweiß-Schiffchen 10 nur nach außen vorsteht, ist am Anschlußport 24, der einerseits auch nach außen vorsteht, ein nach innen in den Beutel hineinragendes Rohr 26 vorgesehen, das den Anschluß eines nach innen ragenden Schlauchs ermöglichen kann. Ein solcher Schlauch kann aber auch direkt an den Port angebracht sein. -

Am Rand des Einschweiß-Schiffchens sind Schweißkanten 28 und 30 vorgesehen, die ein erleichtertes Anschweißen des Beutels an das Einschweiß-Schiffchen sicherstellen.

Ein Einschweiß-Schiffchen gemäß der Figuren 1 bis 3 dient im hier dargestellten Ausführungsbeispiel als Einschweiß-Schiffchen eines hier nicht näher dargestellten Konzentratbeutels, d.h. eines Beutels, der bei der Zubereitung von Dialysierflüssigkeit in einer entsprechenden Dialysiermaschine mit einem Wasserkreislauf verbunden wird. Um dies für den Benutzer zu erleichtern und gleichzeitig das Eindringen von Bakterien zu verhindern, sind die Anschlußports 22 und 24 vorgesehen, die hierfür entsprechend gestaltet sind. Ein Öffnen und/oder Umschütten des Beutels in ein in die Dialysemaschine integriertes Behältnis ist somit nicht notwendig.

Zur Vereinfachung und Automatisierung des Herstellungsprozesses bietet insbesondere die Tülle 18 eine an das Befüllsystem angepaßte Öffnung, die im hier dargestellten Ausführungsbeispiel ca. 35 mm umfaßt. Diese Öffnung kann nach der Befüllung durch einen Verschluß, beispielsweise eine Folie oder einen Hartdeckel, verschlossen werden. Während des gesamten Herstellungsprozesses des Konzentratbeutels kann die Tülle 18 zur Fixierung des Beutels in der automatischen Produktionslinie dienen. Zur Fixierung während der Befüllung und/oder Nutzung können auch weitere Komponenten am Einschweiß-Schiffchen vorgesehen sein.

Die erfindungsgemäße Gestaltung des Einschweiß-Schiffchens mit einer möglichst großen Füllöffnung 16 bei gleichzeitig optimierter Raumnutzung der Fortsätze 14 für die eigentlichen Anwendungsports ist insbesondere bei der Befüllung mit pulverförmigen Produkten vorteilhaft, da diese anders als bei Flüssigkeiten leichter eine zu enge Füllöffnung blockieren können.

In vorteilhafter Weise kann der Beutel vor der Befüllung vollständig gefertigt werden.

Nach der Befüllung läßt sich das Beutelvolumen durch Evakuieren des Beutels über das Einschweiß-Schiffchen minimieren, wodurch zum Beispiel das Konzentratpulver fixiert wird. Das Anlegen des Vakuums kann erfolgen durch Anschluß der Vakuumpumpe an einen der vorhandenen Ports, durch einen separaten Port oder ein Port im Verschluß eines vorhandenen Ports.

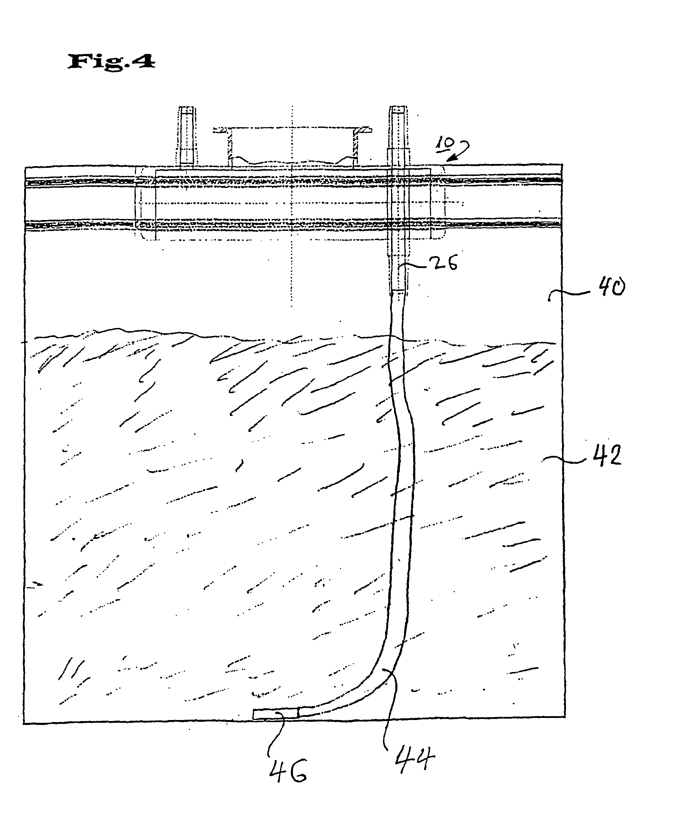

In Figur 4 ist ein Einschweiß-Schiffchen 10 mit einem Beutel 40 verschweißt dargestellt. Der Beutel 40 ist mit Pulver 42 gefüllt, das beispielsweise als Konzentrat zur Herstellung einer Dialyselösung dient. Der in den Beutel hineinragende Port in Form eines Rohres 26 trägt einen Schlauch 44, um einen Zugang zum tiefsten Punkt des Beutels zu erhalten. Im hier dargestellen Ausführungsbeispiel handelt es sich um ein Entnahmeport, so dass der Schlauch an seinem freien Ende zweckmäßigerweise mit einem Filterelement 46 versehen ist, um beim Abgreifen einer gesättigten Lösung am Boden des Beutels Feststoffe am Eindringen in den Schlauch 44 zu hindern. Selbstverständlich könnte auch eine entsprechende Ausführungsform des Beutels dazu dienen Flüssigkeit am Boden des Beutels einströmen zu lassen. In einer derartigen Variante ist ein Filterelement 46 nicht unbedingt notwendig.

## Patentansprüche

1. Einschweiß-Schiffchen (10) für einen Beutel (40) mit einem Mittelteil (12) und zwei sich von diesem aus in entgegengesetzte Richtung erstreckenden spitz zulaufenden Fortsätzen (14), das einerseits mit einer Öffnung (16) zum Befüllen des Beutels mit einer Substanz bei der Herstellung und andererseits mit Zu- und Abgängen (22, 24) für die eigentliche Benutzung des Beutels (40) versehen ist,
**dadurch gekennzeichnet,**
**daß** das Mittelteil (12) im wesentlichen durch die möglichst großlumige, vorzugsweise kreisrunde Öffnung (16) zum Befüllen des Beutels (40) ausgefüllt ist und daß die Seitenkanten der seitlichen Fortsätze (14) sich im Bereich, in dem sie spitz zulaufen tangential an die Öffnung (16) anlegen.

2. Einschweiß-Schiffchen (10) nach Anspruch 1, **dadurch gekennzeichnet, daß** an dessen großlumiger Öffnung (16) eine Tülle (18) angesetzt, vorzugsweise angespritzt, ist.

3. Einschweiß-Schiffchen (10) nach Anspruch 1 oder Anspruch 2, **dadurch gekennzeichnet, daß** im Bereich der Fortsätze (14) Ports (22, 24) bzw. Konnektoren angeordnet sind.

4. Einschweiß-Schiffchen (10) nach einem der Ansprüche 1 bis 3, **dadurch gekennzeichnet, daß** an seinem Umfang mindestens eine Anschweißkante vorgesehen ist.

5. Einschweiß-Schiffchen (10) nach Anspruch 3 oder 4, **dadurch gekennzeichnet, daß** ein Zugangsport in einem Fortsatz (14) und ein Entnahmeport im gegenüberliegenden Fortsatz (14) angeordnet ist.

6. Beutel (40) enthaltend ein Konzentrat, vorzugsweise als Pulver (42), für eine Dialyse-Lösung oder von Teilen davon zur Nierenersatzbehandlung, **gekennzeichnet durch** ein Einschweiß-Schiffchen (10) nach einem der Ansprüche 1 bis 5.

7. Beutel (40) nach Anspruch 6, **dadurch gekennzeichnet, daß** das Einschweiß-Schiffchen (10) einen nur nach außen ragenden rohrförmigen Port und einen in den Beutel (40) hineinragenden und nach außen ragenden rohrförmigen Port (26) aufweist.

## Claims

1. A welding boat (10) for a pouch (40) having a center part (12) and two prolongations (14) extending, starting from this, in opposite directions and tapering acutely, which is provided, on the one hand, with an opening (16) for the filling of the pouch with a substance in manufacture and, on the other hand, with inlets and outlets (22, 24) for the actual use of the pouch (40),
**characterized in that**
the center part (12) is substantially filled by an opening (16) for the filling of the pouch (40) which opening has a lumen which is as large as possible and is preferably circular; and **in that** the side edges of the side prolongations (14) contact the opening (16) tangentially in the region in which they taper acutely.

2. A welding boat (10) in accordance with claim 1, wherein a nose (18) is formed, preferably molded, to its large lumen opening (16).

3. A welding boat (10) in accordance with either of claims 1 or 2, wherein ports (22, 24) or connectors are arranged in the region of the prolongations (14).

4. A welding boat (10) in accordance with any of claims 1 to 3, wherein at least one weld edge is provided at its periphery.

5. A welding boat in accordance with either of claims 3 or 4, wherein an inlet port is arranged in one prolongation (14) and a removal port is arranged in the opposite prolongation (14).

6. A pouch (40) containing a concentrate, preferably as a powder (42), for a dialysis fluid or of parts thereof for kidney replacement treatment, **characterized by** a welding boat (10) in accordance with any of claims 1 to 5.

7. A pouch (40) in accordance with claim 6, wherein the welding boat (10) has one only outwardly projecting tubular port and one tubular port (26) projecting into the pouch (40) and projecting outwardly.

## Revendications

1. Navette soudable (10) pour un sac (40) avec une partie centrale (12) et deux prolongements (14) s'étendant de manière effilée à partir de celle-ci dans la direction opposée, qui est dotée d'une part d'une ouverture (16) pour remplir le sac avec une substance lors de la fabrication, et d'autre part d'amenées et de sorties (22, 24) pour l'utilisation proprement dite du sac (40),
**caractérisée**
**en ce que** la partie centrale (12) est remplie principalement à travers l'ouverture si possible à grand diamètre et de préférence circulaire (16) pour le chargement du sac (40), et en ce que les arêtes latérales des prolongements latéraux (14) sont établies de manière tangentielle à l'ouverture (16), dans la zone dans laquelle elles s'effilent.

2. Navette soudable (10) selon la revendication 1, **caractérisée en ce que** sur l'ouverture à grand diamètre (16) de celle-ci, une douille (18) est appliquée, de préférence injectée.

3. Navette soudable (10) selon les revendications 1 ou 2, **caractérisée en ce que** dans la zone des prolongements (14), des ports (22, 24) et/ou des connecteurs sont disposés.

4. Navette soudable (10) selon une quelconque des revendications 1 à 3, **caractérisée en ce que** sur sa périphérie, au moins une bordure soudée est prévue.

5. Navette soudable (10) selon les revendications 3 ou 4, **caractérisée en ce qu'**un port d'accès est disposé dans un prolongement (14) et un port de prélèvement dans le prolongement se trouvant en face (14).

6. Sac (40) contenant un concentré, de préférence en poudre (42) pour une solution de dialyse ou d'une partie de celle-ci pour le traitement de substitution des reins, **caractérisé par** une navette soudable (10) selon une quelconque des revendications 1 à 5.

7. Sac (40) selon la revendication 6, **caractérisé en ce que** la navette soudable (10) présente un port en forme de tuyau dépassant uniquement vers l'extérieur, et un port (26) en forme de tuyau pénétrant dans le sac (40) et dépassant vers l'extérieur.
